Europäisches Patentamt

(19) European Patent Office

Office européen des brevets

(11) Numéro de publication: **0 207 817**
**B1**

(12) **FASCICULE DE BREVET EUROPEEN**

(45) Date de publication du fascicule du brevet: **02.01.91**

(51) Int. Cl.⁵: **A 61 M 1/00**

(21) Numéro de dépôt: **86400960.0**

(22) Date de dépôt: **02.05.86**

(54) **Tête de raccordement à un dispositif d'accès percutané.**

(30) Priorité: **03.05.85 FR 8506738**

(43) Date de publication de la demande:
**07.01.87 Bulletin 87/02**

(45) Mention de la délivrance du brevet:
**02.01.91 Bulletin 91/01**

(84) Etats contractants désignés:
**AT BE CH DE FR GB IT LI LU NL SE**

(56) Documents cités:
**WO-A-81/03424**
**GB-A-2 050 175**
**GB-A-2 143 133**
**US-A-4 108 174**

(73) Titulaire: **BIOMASYS Société dite:**
**Château de Chailly-sur-Armançon**
**F-21320 POUILLY-EN-AUXOIS (FR)**

(72) Inventeur: **Lapeyre, Didier**
**Chaignes**
**F-27120 Pacy sur Eure (FR)**
Inventeur: **Slonina, Jean-Pierre**
**23 bis, route de la Cascade**
**F-78110 Levesinet (FR)**

(74) Mandataire: **Portal, Gérard et al**
**Cabinet Beau de Loménie 55, rue d'Amsterdam**
**F-75008 Paris (FR)**

Courier Press, Leamington Spa, England.

**Description**

La présente invention concerne un dispositif inamovible de passage percutané pour accès à un circuit de fluides dans un organisme vivant et système de distribution de fluides.

Les fluides qui peuvent être échangés avec un organisme vivant sont le sang mais aussi des produits pharmaceutiques tels que l'insuline, des antibiotiques, des vaso-dilatateurs. L'hémodialyse est un cas important d'échange de sang concerné par le système de l'invention.

Tous ces fluides sont extraits ou injectés dans l'organisme vivant au moyen d'un dispositif d'accès atraumatique inamovible, qui est constitué par un système de valve établissant à volanté une communication entre l'intérieur et l'extérieur de l'organisme vivant. L'implantation d'un tel dispositif d'accès atraumatique se justifie par le traumatisme et la sclérose des tissus vivants que crée le répétition de piquêres par aiguilles hypodermiques lorsque des soins répétés sont obligatoires.

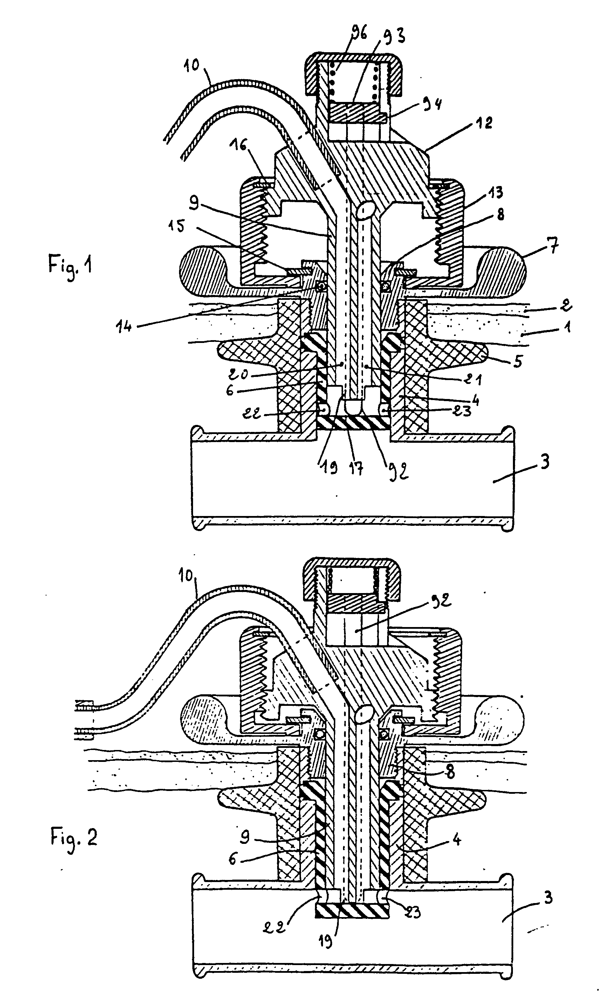

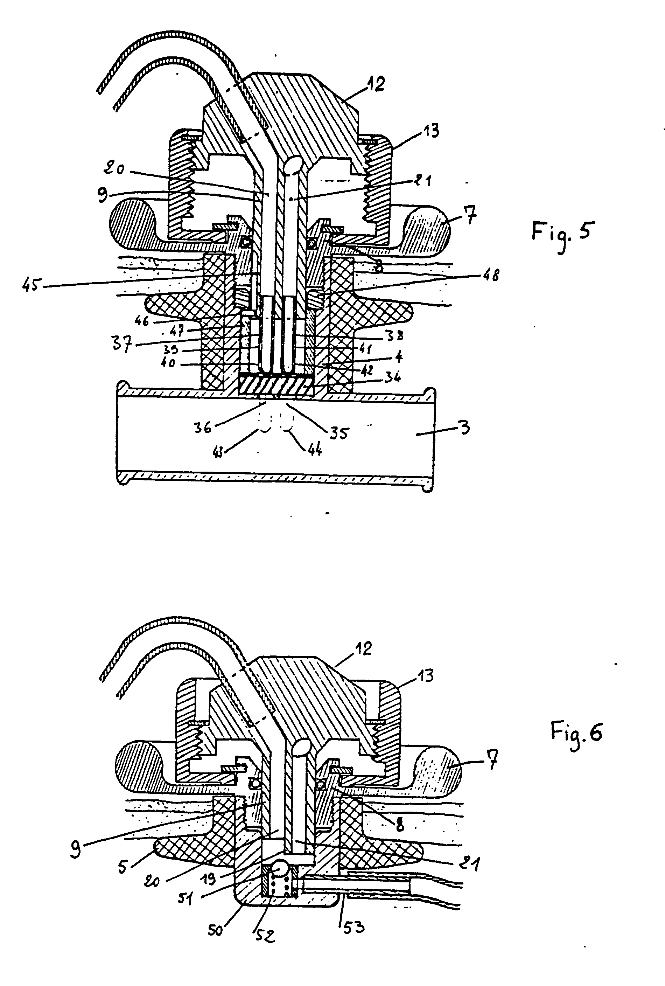

Les dispositif de'accès atraumatique à une circuit interne à l'organisme sont connus. La plupart ont en commun de comporter un corps, dont une partie est en liaison directe avec le circuit de fluide de l'organisme, et dont une partie, isolée de la précédente par une vanne, est en relation avec l'extérieur de l'organisme. Cette seconde partie constitue un puits d'accès au réseau de fluides de l'organisme, tel que le sang, bien que cet exemple ne constitue par un limite à l'application de l'invention; elle traverse le derme, découche au niveau de l'épiderme, et est, en dehors des périodes pendant lesquelles sont pratiqués des échanges de fluide, bouchée par tout moyen étanche aux liquides et aux bactéries. Dans ce puits d'accès est disposée une vanne, formée soit par une membrane extensible, soit par une membrane qui est perçable par une aiguille, soit par une bille ou une soupape mécanique quelconque, soit par un barillet rotatif, soit encore par le corps tronconique d'un robinet "à carotte". Par exemple, le document WI—81/03424 décrit un dispositif d'accès atraumatique sanguin formant valve comportant une partie inamovible formant puits d'accès dans un circuit sanguin (13) dans laquelle est diposé de manière alternativement déplaçable un élément (31) comportant deux canaux longitudinaux (51, 53) complètement indépendants, séparés par une cloison commune, lesdits canaux (51, 53) débouchant à l'extérieur dans la paroi latérale, de manière à être respectivement mis en communication avec le circuit sanguin dans une position enfoncée de l'organe (31), représentée à la figure 1, ou obturés par la paroi interne du dispositif inamovible (7, 15, 5), en position rétracté représentée à la figure 6. Certains de ces systèmes d'accès au réseau sanguin seront exposés ultérieurement.

Quel que soit le type de valve, i¹ demeure toujours une cavité, formée par le puits d'accès, lorsque de bouchon du dispositif atraumatique est enlevé.

On sait que le branchement de tubes, dans lesquels circulent des fluides, sur un dispositif d'accès atraumatique inamovible, conduit à des difficultés de mauvaise désinfection, mauvais rinçage, mauvaise purge de l'air en raison de la manipulation à l'air libre des extrémités ouvertes des tubes eux-mêmes remplis d'air avant l'opération, et en raison de la présence d'un puits d'accès, à l'air.

Jusqu'à présent, l'opération d'asepsie des bouts de tubes et du puits d'accès s'est faite, de façon manuelle, au moyen d'un produit antiseptique, mais comme elle s'effectue à l'air, donc en atmosphère a priori non aseptique, elle est en partie inefficace.

En outre, les produits antiseptiques utilisés dans l'asepsie manuelle sont mal rincés par les produits de rinçage, par exemple à l'intérieur des bouts des tubes, ou dans certaines parties concaves du puits d'accès.

Le problème de la coagulation sanguine lorsqu'il s'agit d'échanges avec le réseau sanguin dans les tubes est partiellement résolu, selon l'art antérieur, par des injections ponctuelles d'héparine, et le débullage est effectué, dans certains systèmes, par au moins un débulleur intégré à l'appareil de traitement, un dialyseur par exemple. Mais ces solutions ne conviennent que pour la partie du système par laquelle du sang est extrait de l'organisme. En ce que concerne la partie par laquelle un liquide, du sang ou un médicament est réinjecté dans le vaisseau sanguin, il y a toujours risque de voir subsister un peu d'air dans le tubes ou dans le puits d'accès, avant que les tubes no soient raccordés sue le dispositif d'accès atraumatique et que la valve no soit ouverte. Il y donc toujours risque d'injecter un peu d'air dans le circuit sanguin, ce qui est très dangereux pour l'organisme.

L'injection, ou l'échange de fluides avec un organisme vivant est donc une opération délicate, présentant des risques, et coûteuse parce qu'elle est réalisée par un personnel hautement qualifié.

C'est donc un objet de la présent invention que de simplifier et rendre fiables les opérations de raccordement d'un dispositif inamovible de passage percutané à une tête de raccordement, reliée par au moins deux tubes à son dispositif d'alimentation en fluides, lui-même relié à un appareil de traitement, tel qu'un hémodialyseur ou un injecteur d'insuline par exemple.

C'est un autre objet de l'invention que d'assurer l'aseptisation, le rinçage et le débullage de toute la partie d'un système comprise entre la valve du dispositif d'accès atraumatique et l'appareil de traitement, la valve étant fermée, avant le début du traitement.

C'est encore un objet de l'invention que de permettre le raccordement de la tête de raccordement sur le dispositif d'accès atraumatique dans des conditions non aseptiques, puis de pratiquer l'asepsie, le rincage et le débullage, sans rompre l'asepsie dans la zone aseptisée, au moyen de commandes externes à cette zone aseptisée, commandes qui peuvent être manuelle mais ont de préférence automatisées.

Ces objectifs sont atteints avec le système selon l'invention, notamment en raison de la conception de la tête de raccordement au dispositif d'accès atraumatique au circuit sanguin. Cette tête comporte un cylindre coaxial au puits d'accès et percé longitudinalement par au moins deux canalisations, ce cylindre étant doté d'au moins un degré de liberté et se déplaçant par translation longitudinale dans une embase fixée sur le dispositif d'accès atraumatique. Dans une première position du cylindre, dans laquelle il est éloigné de la valve, il existe un libre passage pour les fluides entre l'extrémité du cylindre et la valve, ce qui permet la circulation de fluides extérieurs pour aseptiser, rincer et débuller les tubes, le puits d'accès, en particulier les parois internes, et la surface de la valve. Le cylindre est en outre doté, à son extrémité voisine de la valve, de moyens propres à ouvrir cette valve. Ces moyens sont conçus pour interrompre le passage des fluides entre les deux canalisations lorsque lesdits moyens sont en contact avec la valve. Dans une seconde position du cylindre, au contact avec la valve, le cylindre ouvre la valve, et établit la circulation de fluides entre l'organisme vivant et au moins une canalisation forée dans le cylindre sans qu'aucune pièce ait été remise à l'air. Les parties qui ont été aseptisées demeurent donc aseptisées.

En d'autres termes, lorsque le cylindre, dans la tête de raccordement, est en position éloignée de la valve, il y a libre circulation de tous les fluides externes nécessaires à la préparation de l'opération de traitement. Lorsque le cylindre est en position en contact avec la valve, il y a échange de fluides entre l'organisme vivant et l'extérieur, à travers un matériel aseptisé.

Le cylindre est déplacé, par rapport au corps de la tête de raccordement par des moyens mécaniques, fluidiques (air ou liquide) ou électriques.

Des sécurités contrôlent la position de la valve et empêchent toute injection des produits aseptisants dans l'organisme vivant.

Bien entendu l'ensemble de la tête de raccordement et du dispositif d'alimentation en fluides est conçu pour éviter toute cavité, afin d'empêcher la coagulation du sang lorsque ce liquide est concerné.

De façon plus précise, l'invention concerne un dispositif inamovible de passage percutané d'accès au circuit sanguin d'un organisme vivant ou à toute zone de l'organisme, comprenant un puits d'accès formé par un corps creux cylindrique ayant une extrémité interne d'accès au circuit sanguin munie d'une valve ayant une position ouverte en communication avec le fluide sanguin et une position fermée interrompant ladite communication, et une extrémité externe comprenant des moyens de fixation amovible d'une tête de raccordement amovible, ladite tête de raccordement amovible comprenant des moyens d'échange de fluides avec le circuit sanguin comprenant un corps tubulaire percé sur sensiblement toute sa longueur par au moins deux canaux non communiquants au moins lorsque la valve est en position ouverte, des moyens de manoeuvre de ladite valve comprenant un actionneur étant prévus pour amener celle-ci en position ouverte, en communication avec le circuit sanguin, ou en position fermée interrompant ladite communication, une pièce de connexion assurant la connexion entre chacun des canaux et un tube de raccordement extérieur, caractérisé en ce que ledit corps tubulaire est percé sur toute sa longueur par au moins lesdits deux canaux débouchant dans le puits d'accès, lesdits moyens de manoeuvre de la valve laissant libre la circulation des fluides entre les canaux lorsque la valve est fermée et assurant une interruption de la communication entre les canaux lorsque ladite valve est en position ouverte, en communication avec le circuit sanguin, et les moyens de fixation de la tête de raccordement amovible comprennent un embase, dont la partie centrale, en surépaisseur, est vissable sur ledit corps creux et est percée pour former un cylindre de guidage, coaxial avec le corps creux.

Selon une caractéristique avantageuse, ce dispositif inamovible est caractérisé en ce que les moyens de manoeuvre précités sont portés par l'extrémité du corps tubulaire proche de la valve, des moyens d'actionnement de la tête de raccordement amovible communiquant au corps tubulaire au moins un mouvement de translation longitudinale, de sorte que le mouvement de translation du corps tubulaire (9) est capable d'amener la valve de ladite position fermée à ladite position ouverte et de mettre ainsi le circuit sanguin en communication avec des tubes extérieurs.

D'autres variantes de réalisation de dispositif inamovible selon l'invention font l'objet de sous revendications.

L'invention concerne encore un système de distribution de fluides assurant la connexion des fluides entre les tubes extérieurs d'une tête de raccordement et une pluralité de tubes réunis à un appareil de traitement, caractérisé en ce qu'il comprend un dispositif inamovible de passage percutané tel que précédemment defini; ce système peut comprendre avantageusement un boîtier contenant autant de chambres étanches que de tubes qui sont reliés à la tête de raccordement, et chacune de ces chambres étanches possède autant de vannes sans volume mort que de tubes qui y sont raccordés.

L'invention sera mieux comprise par la description détaillée de quelques exemples de réalisations, cette description s'appuyant sur les figures en annexe qui représentent:

figure 1: vue en coupe d'un premier exemple de dispositif inamovible de passage percutané selon l'invention, en position de valve fermée,

figure 2: vue en coupe du dispositif de la figure 1, la valve étant en position ouverte,

figure 3: vue en coupe du dispositif selon un plan orthogonal à celui de la figure 2, la valve étant en position fermée,

figure 4: vue en plan du dispositif de la figure 1,

figure 5: vue en coupe de premier exemple du dispositif selon l'invention, adapté à un deuxième type de valve de passage percutané, la valve étant en position fermée,

figure 6: vue en coupe du premier exemple du dispositif selon l'invention, adapté à l'injection de fluides médicamentaux dans l'organisme vivant, valve en position fermée,

figure 7: schéma d'adaptation d'un actionnement électrique du premier exemple du dispositif, selon l'invention,

figure 8: vue en coupe d'un deuxième exemple du dispositif selon l'invention, dans le cas d'un actionnement fluide par air ou liquide,

figure 9: schéma du raccordement entre les canaux intérieurs et les tubes extérieurs, dans un troisième exemple du mole de connexion de la pièce 9, le type d'actionnement fluidique, mécanique ou électrique n'étant pas représenté,

figure 10: schéma du raccordement entre les canaux intérieurs et les tubes extérieurs, dans un quatrième exemple du mode de connexion de la pièce 9, le type d'actionnement, mécanique ou électrique n'étant pas représenté,

figure 11: vue en plan, correspondant à la figure 8,

figure 12: vue en élévation de la forme des gorges de raccordement dans le cas de deuxième exemple du dispositif selon l'invention,

figure 13: schéma d'adaptation d'un actionnement mécanique dans le cas du deuxième exemple du dispositif selon l'invention,

figure 14: schéma d'adaptation d'un actionnement électrique dans le cas du deuxième exemple du dispositif,

figure 15: vue en coupe du deuxième exemple du dispositif dans le cas d'un actionner fluidique, figure 8 montrant un système se sécurité sur la circulation de fluides dangereux pour l'organisme vivant,

figure 16: vue en plan du système de sécurité de la figure précédente,

figure 17: schéma simplifié d'un système de traitement comportant un dispositif inamovible de passage percutané et un boîtier de distribution des fluides selon l'invention, et

figure 18: vue en coupe, schématique, d'un boîtier de distribution sans volumes morts.

La figure 1 représente la vue en coupe d'un premier exemple de réalisation d'un dispositif amovible selon l'invention, comprenant un disposifit d'accès atraumatique connu, décrit dans le demande de brevet FR—A—2561922 qui a été déposée le 2 avril 1984 par la demanderesse (EP—A—0159260).

L'invention sera mieux comprise par le rappel préliminaire de la structure et du fonctionnement d'un dispositif d'accès atraumatique à un circuit de fluide dans un organisme vivant, qui sera désigné par "passage percutané" inamovible dans la suite du texte, afin de simplifier le langage. Dans le même esprit, on considérera un circuit sanguin, que est un cas de circuit de fluide dans un organisme vivant.

Le passage percutané est implanté dans l'organisme, sous le derme 1 et l'épiderme 2. Il comporte essentiellement un té de dérivation, dont les deux parties alignées forment un tube 3, aux extrémités duquel sont fixées de courtes canalisation sanguines artificielles souples suturées respectivement à une veine et une artère. Tout cela dans le cas, non limitatif du shunt artérioveineux.

La troisième partie de té forme un corps creux 4, qui relie la surface de l'épiderme 2 au tube 3 parcouru par le sang. Afin de permettre l'ancrage du passage percutané dans les tissus de l'organisme, le corps creux 4 est solidaire d'une bague 5, munie d'au moins une collerette. La bague 5 est réalisé en fibres de carbone imprégnées de carbone, ce qui lui confère une structure poreuse: les tissue en colonisant les pores de la bague 5 assurent la fixation du passage percutané dans l'organisme, le rendant ainsi inamovible.

La valve qui commande les échange de sang ou de fluides entre l'intérieur de l'organisme vivant et l'extérieur est ici constituée par un corps 6, en forme de piston à l'intérieur du corps creux 4, avec lequel il assure une bonne étanchéité. Ce piston 6 est réalisé en matériau élastomère, et est fixé par l'une de ses extrémités dans le corps creux 4, cependant que son autre extrémité, fermée, est au niveau de la paroi du tube 3, mais ne pénètre pas dans le tube 3 lorsque la valve est fermée. Ainsi, la paroi interne du corps creux 4 obstrue deux ouvertures 22 et 23 pratiquées dans la paroi cylindrique du piston 6.

Pour ouvrir la valve, il faut déformer le piston en élastomère 6, en l'allongeant. Son extrémité proche du tube 3 pénètre alors dans celui-ci, ce qui a pour effet de libérer les ouvertures 22 et 23, et d'établir une circulation de sang ou de fluides entre les vaisseaux sanguins, par l'intermédiaire du tube 3, et l'extérieur de l'organisme. Des moyens appropriés permettent de séparar les flux, et d'utiliser une ouverture, 22 par example, pour extraire du sang, et l'autre ouverture, 23 dans ce cas, pour réinjecter du sang et/ou des liquides pharmaceutiques. La valve est représentée ouverte en figure 2.

Le corps creux 4 comporte enfin, du côté de son extrémité externe à l'organisme, un filetage qui permet de fixer soit un bouchon lorsque le passage percutané est inutilisé, soit une tête de raccordement lorsqu'il est utilisé pour des échanges de fluides avec le réseau sanguin.

Le passage percutané présente donc un puits d'accès, constitué par l'intérieur du corps creux 4 et du piston 6: ce puits d'accès doit être désinfecté et rincé avant l'opération de traitement du sang. C'est des fonctions remplies par le dispositif selon l'invention représentée en figure 1.

Le dispositif comprend essentiellement une embase 7 fixable sur le corps creux 4 du passage percutané. Tout les moyens de fixation mécanique connus, sont utilisables pour fixer l'embase 7 sur le corps 4, pourvu qu'ils offrent une bonne étanchéité et une position repérée de l'embase par rapport au corps, mais un filetage est le moyen préféré comme étant le moins traumauti- sant pour le patient et celui qui exerce le moins d'efforts sur le passage percutané.

Cette embase comprend sur son pourtour une collerette qui sert à la visser dans le corps creux 4, et, dans sa partie centrale, une surépaisseur percée en son centre et coaxialement avec le corps creux 4. Ce passage est usiné avec précision, en sorte qu'il constitue un cylindre de guidage 8. De plus, il a un diamètre intérieur égal au diamètre intérieur du piston 6 en élastomère.

Dans le cylinder de guidage 8 est ajusté un corps tubulaire 9, qui peut se déplacer selon au moins un degré de liberté, selon l'axe commun au corps creux 4, au piston 6 et au cylindre de guidage 8. Ce corps tubulaire 9 est percé longitudinalement de préférence selon un diamètre, d'au moins deux canaux 20 et 21. En effet, même dans le cas où le passage percutané est utilisé pour seulement injecter un produit dans le sang, un second canal est nécessaire pour assurer une circulation des liquides aseptisants et de rinçage, ainsi que le débullage, au cours des opérations préliminaires.

Les canaux 20 et 21 communiquent à une extrémité du corps tubulaire 9 avec le matériel de traitement par l'intermédiaire de tubes 10 et 11. Ils débouchent à l'autre extrémité du corps tubulaire 9. Ladite extrémité porte des moyens de manoeuvre de la valve 6, sous la double condition de laisser libre la circulation des fluides entre les canaux 20 et 32 lorsque la valve est fermée, et d'isoler les mêmes canaux lorsque la valve est ouverte. Ces moyens sont variables selon le type de valve utilisé.

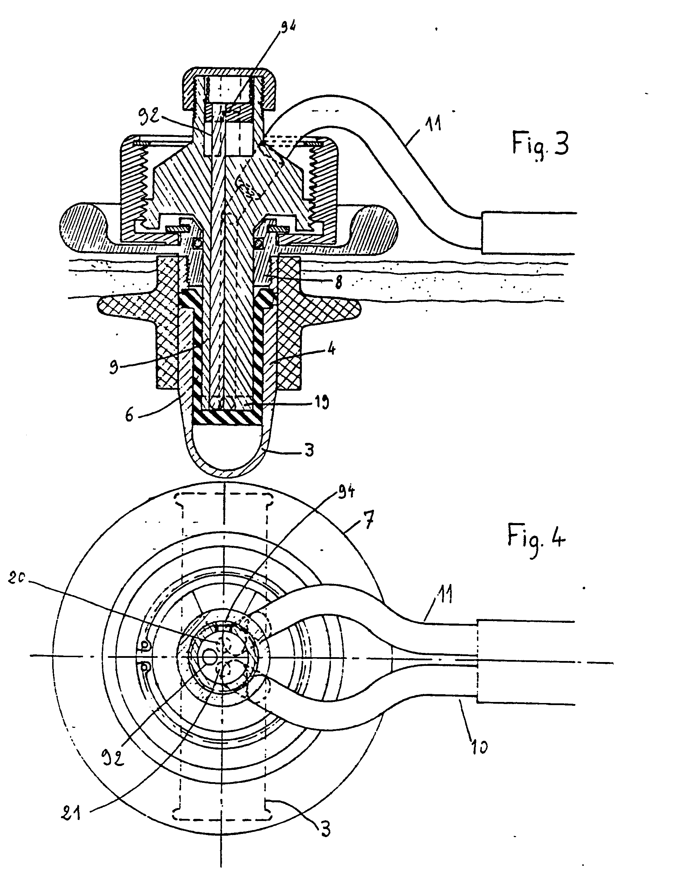

Dans le cas de valve conforme à la demande de brevet FR—A—2561922 de la demanderesse, valve qui nécessite une élongation pour être ouverte, le moyen de l'ouvrir est constitué par une saillie 19, solidaire du corps tubulaire 9. Cette saillie 19 a une longueur — mesurée selon l'axe de déplacement du corps tubulaire 9 — sensiblement égale au diamètre des ouvertures 22 et 23 du piston 6 en élastomère, de façon à permettre un écoulement régulier du sang entre les ouvertures 22 et 23 et les canaux 20 et 21, respectivement, sans risque de coagulation du sang dans une cavité non balayée par le flux sanguin, comme cela ressort sur la figure 2. De plus, la saillie 19 est constituée, dans le présent cas de valve élastomère, par une portion du corps tubulaire 9 formant lame le long d'un diamètre perpendiculaire au diamètre portant les deux canaux 20 et 21. Les figures 1 et 2 montrent cette lame selon son épaisseur, tandis que la figure 3 la montre selon sa largeur c'est-à-dire dans l'axe des deux ouvertures 22 et 23.

L'étanchéité aux fluides et aux bactérie de l'ensemble passage percutané — tête de raccordement est assurée par:

—l'insertion légèrement à force du piston 6 dans le corps creux 4, et son maintien au moyen d'une gorge.

—l'adaptation à frottement doux entre la surface extérieure du corps tubulaire 9 et la surface intérieure du piston 6.

—un joint d'étanchéité 14 entre le cylindre de guidage 8 et le corps tubulaire 9.

—l'appui de la pièce 7 sur le sommet du cylindre 4.

La fonction et l'usage d'autres parties représentées sur les figures 1, 2 et 3 seront explicitées et développées ultérieurement, ainsi que l'adaptation de la tête de raccordement à d'autres types de valves.

La fonctionnement du dispositif selon l'invention se comprend facilement, en regard des figures 1, 2 et 3.

Sur la figure 1, le corps tubulaire 9 est en position éloignée de la valve 6: pour simplifier le langage, cette position sera appelée position "haute". Il existe une communication entre les canaux 20 et 21, à l'intérieur du piston 6, car la lame 19 n'entre pas en contact avec la paroi plane du piston 6. La valve est donc fermée. Il est alors possible d'introduire par un tube, 10 par exemple, un fluide aseptisant, puis de rincer au moyen d'eau distillée ou de sérum physiologique. Le débullage se fait simultanément, et tous ces fluides, ainsi que les bulles, sont extraits par le second tube, 11 dans ce cas. L'ensemble des pièces qui n'étaient pas aseptiques, et qui vont être ultérieurement en contact avec le sang, est ainsi aseptisé.

En figure 2, et sans que la tête de raccordement ait été démontée ou mise à l'air, le corps tubulaire 9 a subi une translation le long de son axe; il est en contact avec la valve 6, qu'il allonge. Cette position sera appelée position "basse".

Le moyen par lequel le corps tubulaire 9 a subi une translation sera exposé ultérieurement. Dans cette position basse, la valve étant ouverte, les ouvertures 22 et 23 du piston 6 sont en communication avec les canaux 20 et 21, respectivement, tandis que la lame 19 assure l'étanchéité avec le font et la paroi interne du piston 6, ainsi que le montre la figure 3. Dans ce cas, le sang peut sortir du passage percutané par exemple à travers l'ouverture 22 et le canal 20, et être réinjecté, après traitement, à travers le canal 21 et l'ouverture 23.

A la fin du traitement, le corps tubulaire 9 est ramené en position haute, ce qui referme la valve 6 et interrompt l'échange sanguin. L'ensemble des tubes et de la tête de raccordement peut alors être balayé du sang, et lavé par des fluides adéquats puisque la communication entre les canaux 20 et 21 est rétablie. Après rinçage, à la fin du traitement, la tête de raccordement est dévissée, et le passage percutané est bouché par un bouchon fileté.

La figure 4 complète les figures en coupe 1 à 3:

Sa vue en plan de dessus permet de mettre en évidence les deux tubes extérieurs 10 et 11, qui mettent en communication les canaux 20 et 21, forés dans le corps tubulaire 9, avec la partie initiale du système de traitement, cette dite partie initiale étant constituée par un boîtier d'alimentation en fluides divers—le terme alimentation étant pris dans son sens le plus général, c'est-à-dire aussi bien arrivées qu'évacuations de fluides.

La figure 5 représente une forme d'adaptation du dispositif selon l'invention à un deuxième type de valve de passage percutané connu.

Cette valve est constituée par une membrane épaisse 34 en élastomère, maintenue contre la paroi du tube 3 de dérivation sanguine au moyen d'un manchon 47, concentrique au corps creux 4, et d'une bague filetée 48. La membrane 34 est percée d'au moins deux séries de fentes, en étoiles, pratiquées selon les axes 35 et 36. En l'absence de toute contrainte, les lèvres des fentes se refermant, et la membrane 34 est étanche, aux faibles pressions considérées: la valve est fermée.

Pour ouvrir la valve, deux aiguilles hypodermiques ou tubes 37 et 38 sont poussés à travers le membrane 34, dont les fentes s'ouvrent par élasticité. A la position des fentes correspondent des ouvertures dans la paroi du tube 3. De préférence, pour éviter le bouchage par des particules élastomères, les tubes sont arrondis à leur extrémité, et ils comportent une série d'ouvertures telles que 39 et 40, 41 et 42 le long d'une génératrice. La figure 5 représente la valve en position fermée, et la tête de raccordement en position haute. Lorsque la valve est ouverte, et la tête de raccordement en position basse, les tubes viennent dans la position 43—44 représentée en pointillé.

Le dispositif selon l'invention comporte toujours une embase 7, fixable sur le té de dérivation sanguine et une partie en surépaisseur 8 formant cylindre de guidage, dans lequel se déplace un corps tubulaire 9 foré longitudinalement par deux canaux 20 et 21.

Les tubes 37 et 38, qui font partie intégrante de la valve, sont fixés dans le canaux 20 et 21, par tout moyen convenable.

Le moyen qui permet d'ouvrir la valve est constitué par le corps cylindrique 9 lui-même, qui pousse les tubes 37 et 38 à travers la membrane 34.

Le moyen qui assure l'interruption de la circulation de fluides, entre les canaux 20 et 21, lorsque la valve est ouverte, est constitué par l'extrémité inférieure plane du corps cylindrique 9, qui vient en contact avec la surface plane de la membrane 34.

Le fonctionnement du dispositif selon l'invention est identique dans le cas de la figure 5 à celui des figures précédentes. En position haute, valve fermée, les fluides antiseptiques et de rinçage balaient, et débullent, les tubes extérieurs, un premier canal 20, en sortant par les ouvertures 39, 40, balaient le volume intérieure au manchon 47, et ressortent par le canal 21, via les ouvertures 41 et 42. Toutes les surfaces sont donc aseptisées et rincées. La valve est ouverte, sans qu'aucune pièce ne soit remise à l'air, par une translation vers la position basse du corps cylindrique 9: le sang peut alors sortir de l'organisme par exemple par les ouvertures 39, 40, le tube 37 et le canal 20, et être réinjecté après traitement par le canal 21, le tube 38 et les ouvertures 41, 42.

Contrairement aux autre cas décrits pour lesquels la position angulaire de la pièce 9 demande un positionnement très approximatif qui se réalise en orientant à peu près les tubes 10 et 11 à l'extérieur, le cas de la figure 5 demande une orientation précise de la pièce 9. Il faut en effet que les tubes 37 et 38 passent exactement par les axes 35 et 36. On y parvient grâce à la rainure 45 et l'ergot 46.

La figure 6 représente une autre adaptation du dispositif selon l'invention à un troisième type de passage percutané à une seule voie, destinée par exemple à injecter à intervalles réguliers un médicament dans l'organisme, tel que de l'insuline.

Le passage percutané est dans ce cas modifé par rapport à ceux qui ont été exposés jusqu'à présent. Une collerette 5, en composite carbone-carbone, fixe sous le derme 1 et l'épiderme 2 un corps creux 50, dont le fond comprend une valve fermée par une bille 51, poussée contre son siège par un ressort 52. Cette valve, normalement fermée en l'absence de toute contrainte extérieure, débouche sur une canalisation 53 raccordée à un vaisseau sanguin par exemple. Le corps creux 50 comporte, de même que le corps creux 4 situé sur les tés de dérivation sanguine, un filetage qui permet d'y fixer soit un bouchon soit une tête de raccordement.

Le dispositif selon l'invention a toujours la même structure qui ne sera plus détaillée: embase 7 et cylindre de guidage 8, corps tubulaire 9 foré longitudinalment par deux canaux 20 et 21.

Le moyen de manoeuvre de la valve est, comme dans les cas des figures précédentes, constitué par une saillie 19, solidaire du corps tubulaire 9. Le matériau qui forme cette saillie entoure complètement un canal, 21 par exemple, de sorte que celui-ci se trouve prolongé par rapport à l'autre canal. De plus, cette saillie 19 est partiellement à l'aplomb de la bille 51: lorsque le corps cylindrique 9 est en position basse, la saillie repousse la bille 51 et le ressort 52 et la valve est ouverte.

Le moyen qui interrompt la circulation des fluides entre les canaux 20 et 21 est également la saillie 19 qui entoure l'un des deux canaux, 21 par exemple. En d'autres termes, le canal 21 est plus long que le canal 20, et se termine selon une surface plane et parallèle à la surface du fond du corps creux 50. Lorsque le corps cylindrique 9 est en position basse, l'extrémité du canal 21 est en contact continu avec la surface du fond du corps creux 50, et le canal 21 est obturé.

On comprend aisément que, lorsque le corps cylindrique 9 est en position haute, la valve est fermée, et la communication entre les canaux 20 et 21 permet l'aseptisation et le rincage de l'ensemble des surfaces préalablement non aseptiques. Lorsque le corps cylindrique 9 est en position basse, l'un des deux canaux, 21 dans l'exemple, est obturé, la valve est ouverte, et l'injection d'un produit pharmaceutique dans le circuit sanguin peut s'effectuer. Jusqu'à présent, les moyens par lesquels la tête de raccordement est actionnée, et le corps cylindrique 9 animé d'un mouvement de translation le long de son axe principal, n'ont pas été expliqués. Ces moyens sont multiples.

Revenant sur les figures 1, 2 et 3, on voit représenté un premier moyen mécanique et manuel de translation longitudinale du corps cylindrique 9.

Celui-ci comporte du côté de son extrémité externe à l'organisme vivant une pièce 12 de forme adaptée pour assurer la connexion des canaux intérieurs 20 et 21 avec des tubes extérieurs 10 et 11, au moyen de deux embouts métalliques.

Cette première pièce 12 de connexion entre tubes, qui est donc solidaire du corps cylindrique 9 et d'ailleurs usinée dans le même bloc de matériau, comporte, sur sa circonférence de plus grand diamètre, un filetage auquel correspond un écrou de manoeuvre 13.

Par rotation de l'écran de manoeuvre 13, la pièce de connexion 12 se déplace le long du filetage, imprimant là un mouvement de translation au corps cylindrique 9. L'écrou de manoeuvre 13 a une position fixe, par rapport à l'axe longitudinal de la tête de raccordement, parce qu'il s'appuie sur l'embase 7 et qu'une rondelle ou circlip 15 l'empêche de translater le long de cet axe.

La longueur du filetage de l'écrou de manoeuvre 13 est égale à la longueur de la translation possible du corps cylindrique 9, sans détérioration de la valve 6 en élastomère.

Une autre rondelle ou circlip 16 empêche la pièce de connexion 12 de sortir du filetage.

Bien entendu, tout moyen mécanique autre que celui représenté sur les figures 1 à 3 appartient au domaine de l'invention. Par exemple, le mouvement de translation vers la position dite haute (valve fermée) peut être créé par un ressort à boudin, prenant appui sur les faces en regard de l'embase 7 et de la pièce de connexion 12, tandis que le mouvement de translation vers la position basse (valve ouverte) résulte de l'action d'une came prenant appui sur un étrier qui remplace l'écrou de manoeuvre 13, et contrecarre l'action du ressort, en poussant sur la pièce de connexion 12.

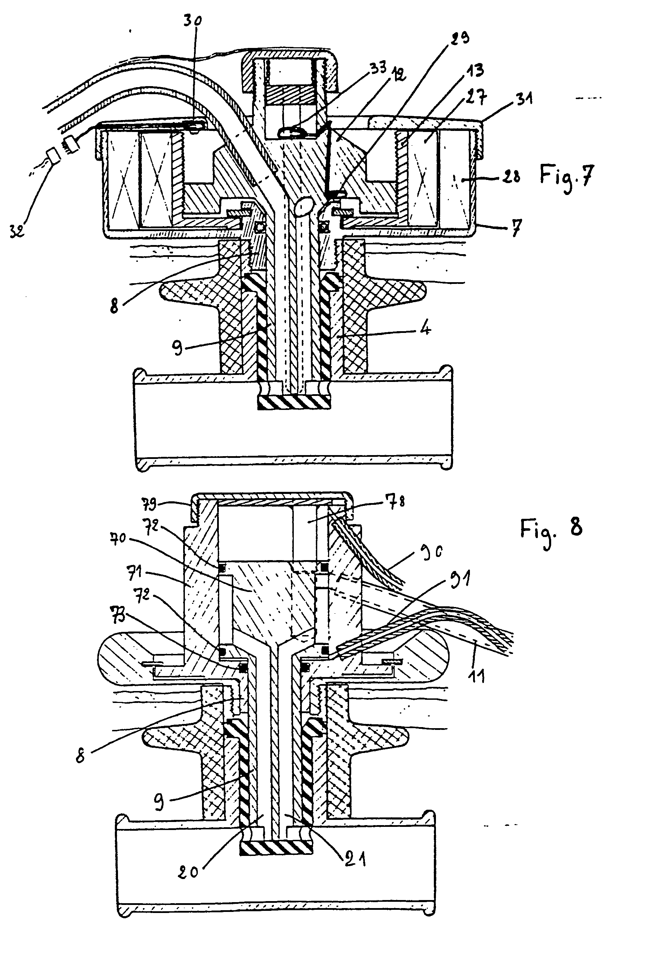

La figure 7 représente un autre moyen d'actionnement du dispositif selon l'invention. Il s'agit d'un moteur électrique qui est de préférence commandé par un séquenceur d'un appareil d'automatisation des opérations de traitement.

L'embase 7 de la tête de raccordement est, dans ce cas, de forme légèrement différente, et elle supporté, sur sa couronne extérieure, un stator 28 de moteur électrique, alimenté par des fils conducteurs au moyen d'un connecteur 32.

L'écrou de manoeuvre 13 a, lui aussi, une forme adaptée et il est solidaire d'un rotor 27, formant, avec le stator 28, le moteur électrique. Le filetage porté par l'écrou de manoeuvre 13 est dans ce cas d'un pas plus petit que le pas du moyen mécanique, afin de servir de démultiplication pour que le mouvement de translation ne soit pas brutal.

La pièce de connexion 12 est de même type que celle du moyen mécanique, mais son filetage est bien entendu adapté à celui de l'écrou de manoeure 13.

Deux contacts électriques 29 et 30, placés en fin de course de la pièce de connexion 12, contrôlent le déplacement du corps cylindrique 9: lorsque la pièce de connexion écrase l'un de ces contacts 29 ou 30, elle interrompt de ce fait le passage du courant électrique dans le moteur 27+28, qui est bien entendu à double sens de rotation. Le contact 30 de fin de course en position haute peut par exemple être porté par le couvercle 31 du boîtier du moteur.

Le palier de roulement du rotor 13+27 peut être très rudimentaire et constitué par le frottement de la pièce 13 en Teflon P.T.F.E. sur la pièce 7 métallique. On pourrait cependant utiliser au même endroit un roulement à billes.

La figure 8 représente une deuxième exemple de réalisation du dispositif selon l'invention qui est également utilisé dans les figures 11, 12, 13, 14, 15 et 16. Cette figure 8 décrit un actionnement par pression de fluides (air ou liquides).

La têté de raccordement comprend toujours, une embase 7, nécessaire à la fixation de la tête sur le passage percutané, mais le cylindre de guidage 8 est dans ce cas solidaire d'un cylindre 71 de l'actionneur fluidique. Le corps tubulaire 9 est, lui, solidaire d'un piston 70, qui se déplace dans le cylindre 71 de l'actionneur, fermé par un couvercle 79.

Les fluides moteurs—air ou liquides—sont introduits par des tubulures 90 et 91 et agissent, respectivement, entre la face supérieure du piston 70 et le couvercle 79, dont le filetage comporte un produit d'étanchéité, et entre la face inférieure du piston 70 et la face supérieure du cylindre de guidage 8.

Des joints 72, sur le piston 70 de l'actionneur, et 73 sur le cylindre de guidage 8, empêchent les fluides moteurs de se mélanger aux fluides sanguins ou pharmaceutiques qui traversent la tête de raccordement.

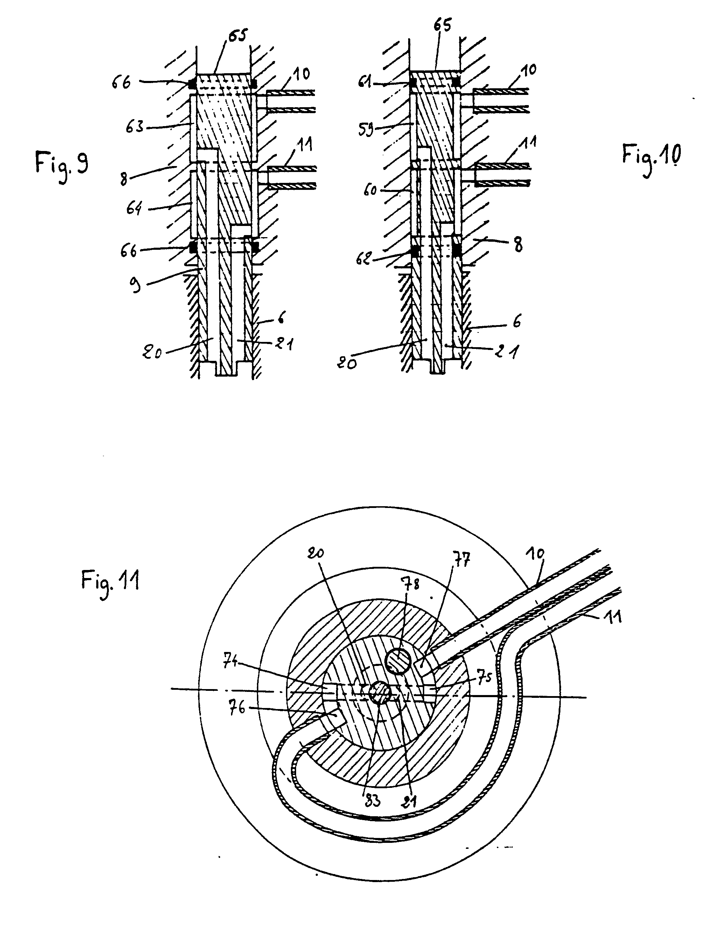

Du fait que la pièce 12 de connexion aux tubes extérieures 10 et 11 est, dans le cas de la figure 8, c'est-à-dire du deuxième exemple de la tête de raccordement selon l'invention, complètement entourée par le cylindre 71 de l'actionneur fluidique, il est nécessaire d'adapter en conséquence la connexion des canaux 20 et 21 aux tubes extérieurs 10 et 11. La figure 8 en montre une exemple, qui n'est pas limitatif. Les figures 9 et 10 qui sont d'autres exemples de réalisation du mode de connexion de la pièce 9 indiquent en effet d'autres possibilités. Sur celle-ci, seules les pièces concernées par la connexion des fluides, sont représentés, de façon à simplifier les figures. Les moyens d'actionnement pourraient être conformes à ceux déjà décrits.

La figure 9 représente le corps tubulaire 9, une partie du corps de soupape 6 en élastomère et une partie du cylindre de guidage 8. Celui-ci forme cylindre en contact à frottement doux avec le corps tubulaire 9. Dans la paroi interne du cylindre de guidage 8 sont pratiqués deux évidements cylindriques 63 et 64, qui sont en communication, à leur première extrémité, avec les canaux 20 et 21, respectivement, et à leur seconde extrémité avec les tubes extérieurs 10 et 11 respectvement. La longueur des rainures 63 et 64 est égale à la longueur de la translation du corps tubulaire 9, et leur section est en accord avec la section des canaux 20 et 21, ou des tubes 10 et 11, de façon à ne pas présenter de résistance du passage du sang. Ces rainures 63 et 64 permettent donc un écoulement continue des fluides depuis le passage percutané vers les tubes extérieurs 10 et 11 quelle que soit la position, haute ou basse, du corps tubulaire 9. Des joints racleurs 66, situés au-dessus et au-dessous des deux rainures assurent l'étanchéité des deux connexions. La face supérieure 65 du corps cylindrique 9 peut être en relation, par tout moyen connu de l'homme de l'art, soit avec un actionneur mécanique, soit avec un actionneur électrique, soit avec un actionneur fluidique.

La figure 10 représente une variant de ce procédé de raccordement par rainures, mais, tandis, que dans l'exemple de la figure 9, le corps tubulaire 9 a une paroi externe unie et les évidements cylindriques sont creusées dans le cylindre de guidage 8, selon cette variante, c'est le cylindre de guidage 8 qui a une paroi interne unie, et les évidements cylindriques 59 et 60 sont creusés dans le corps tubulaire 9. Des joints 61 et 62 assurent l'étanchéité entre les pièces traversées par le sang et l'extérieure de la têté de raccordement.

Eventuellement, une légère fuite qui existerait entre les rainures 59 et 60, ou 63 et 64, par suite d'un ajustage insuffisant entre corps tubulaire 9 est cylindre de guidage 8, n'aurait pas de graves conséquences: c'est le même fluide, sang par exemple, qui circule dans ces rainures.

On notera que, dans le cas des figures 9 et 10, les évidements circulaires 59, 60, 63, 64 dans le piston ou le cylindre pourraient être des rainures parallèles à l'axe du piston mais que, dans ce cas, il faudrait une immobilisation en rotation du type de l'ergot coulissant 78 figure 8.

Les figures 9 et 10 sont en fait des figures de généralisation. En réalité, et est déconseillé que les évidements cylindriques 59 et 60 ou 63 et 64 soient toriques et coaxiaux au corps tubulaire 9, car cela risque d'entraîner un mauvais balayage par le fluide qui les traverse, entre l'orifice d'un canal, 20 ou 21, et l'orifice d'un tube, 10 ou 11, et coagulation du sang stagnant.

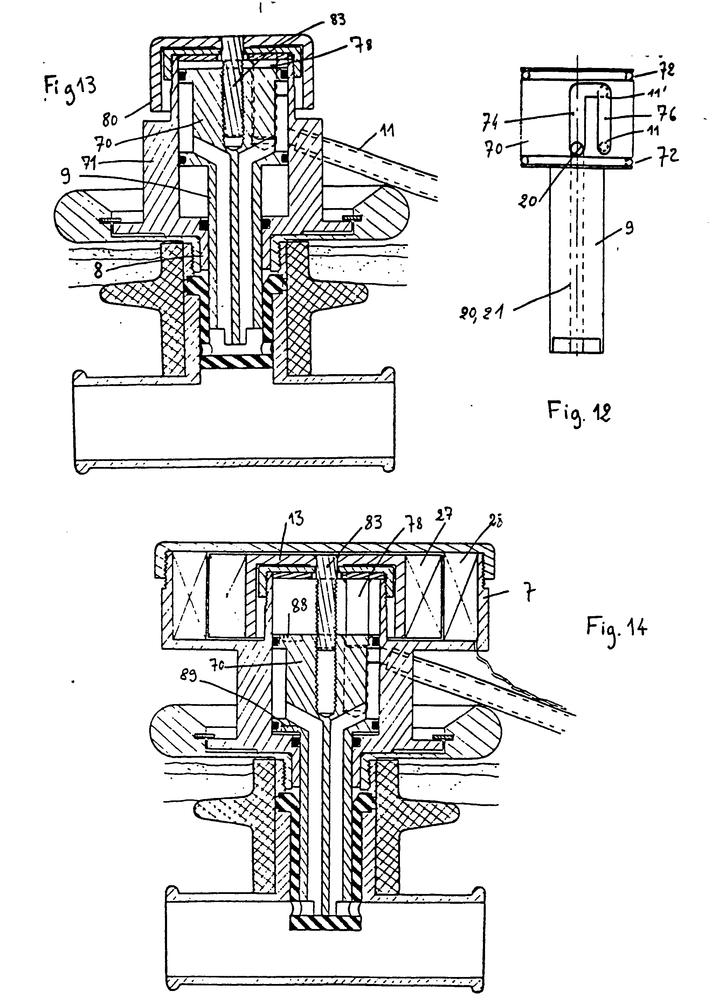

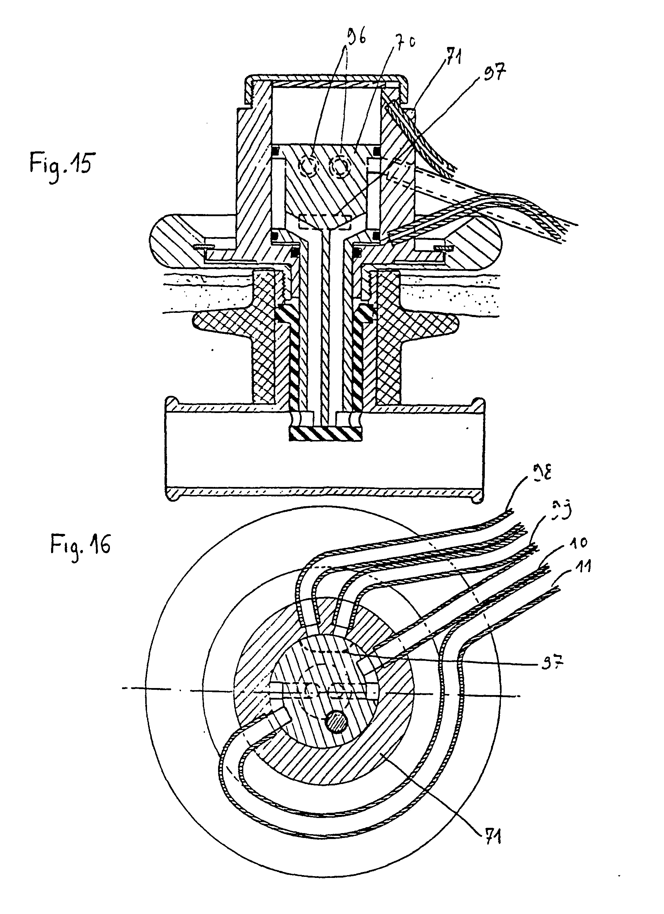

C'est pourquoi ces rainures ont une forme en U, qui ressort des figures 11, 12 et 13. La figure 11 et une vue de dessus, en coupe, de la tête de raccordement de la figure 13, tandis que la figure 12 est une vue en élévation du corps tubulaire 9, montrant l'une des rainures en U, l'autre étant symétrique et cachée par le corps.

La figure 11 met en évidence que les rainures 74—76 et 75—77 n'entourent pas complètement le corps tubulaire 9. Par contre, elles sont au même niveau et de préférence diamètralement opposées.

La figure 12 montre la forme en U d'une rainure. Chaque rainure 74—76 ou 75—77 se décompose en une partie longitudinale 74, à la base de laquelle débouche un canal, 20 par exemple, puis une partie transversale 79, qui communique avec une seconde partie longitudinale 76. Les deux parties 74 et 76 ont une longueur égale à la longueur de translation du corps cylindrique 9. Le tube extérieur 10 pour la rainure 75—77 et le tube 11 pour la rainure 74—76 débouchent dans des rainures 77 et 76 respectivement.

Lorsque la valve 6 est fermée et le corps tubulaire 9 en position haute, le tube extérieur 11 débouche dans la partie basse de la rainure 76: le fluide aseptisant et le fluide de rinçage balayant la totalité des rainures 74, 79, 76, et les débullant.

Lorsque le corps tubulaire 9 est en position basse, et la valve 6 ouverte, le tube 11 débouche, voir figure 12, dans la partie haute de la rainure 76 en 11': le sang circule dans les rainures 74 et 79. La rainure 76 constitue dans ce cas un cul-de-sac, mais cela ne présente pas d'inconvénient puisque la dernière opération avant d'introduire du sang a été de rincer au sérum physiologique: la rainure 76 est donc remplie de sérum physiologique, sans danger pour l'organisme et ne risquant pas la coagulation.

Cette forme de rainure explique et justifie la forme du corps tubulaire et de sa pièce de connexion 70, sur la figure 8, dotée d'un actionneur fluidique ou sur les figures 11 à 16.

La figure 13 représente par ailleurs comme la figure 8 un dispositif selon l'invention dans le deuxième exemple de réalisation mais, dans le cas de la figure 13, le mode d'actionnement est mécanique au lieu d'être hydraulique.

Etant donné que les tubes extérieurs 10 et 11 ne sont plus raccordés sur l'extrémité du corps tubulaire 9, par l'intermédiaire d'une pièce de raccordement 12, comme cela était le cas en figure 1, il devient possible d'installer un actionneur mécanique axial, manuel ou automatisé. Celui-ci comporte un écrou de manoeuvre 80, doté d'un axe fileté 83 qui coopère avec un filetage dans la pièce de connexion 70. Un couvercle vissé sur la figure 71 immobilise longitudinalement l'écrou de manoeuvre 80 grâce à une collereté située sur la base de la tige filetée 83. Cette tige 83 est soudée sur l'écrou 80 après avoir emprisonné le couvercle. On visse sur la pièce 71 en utilisant des trous de passage non représentés sur le sommet de l'écrou 80.

Une butée 78 coulissant dans la pièce 70, représentée également sur les figures 8 et 11, empêche le corps tubulaire 9 de tourner lorsque l'écrou de manoeuvre 80 est vissé ou dévissé et maintient toujours le canal 11 en face de la rainure 76 et le canal 10 en face de la rainure 77.

La figure 14 représente une autre adaptation de la même tête de raccordement à un actionneur électrique. La tête est cette fois-ci en position basse, valve ouverte.

Le moteur électrique est en tout point comparable au moteur de la figure 7, mais le rotor 27 agit directement sur l'axe fileté 83. La course du corps tubulaire 9 est arrêtée lorsque l'un des contacts électriques—non représentés—est actionné par l'une ou l'autre face plane du piston 70, das les régions 88 ou 89 par exemple qui sont des évidements contenant ces contacts électriques.

Le dispositif selon l'invention comporte encore des sécurités destinées à empêcher toute fausse manoueuvre préjudiciable à l'organisme vivant. Leur exposé nécessite de revenir sur les figures précédentes.

Les figures 1 à 4 montrent une sécurité de position de valve. Etant donné que la valve 6 en élastomère est à l'intérieur du passage percutané et cachée par la tête de raccordement de l'invention, on ne voit pas si la valve est en position fermée ou, par suite d'un incident, bloquée en position ouverte ou même rompue. Afin d'éviter l'injection de fluides aseptisants dans l'organisme vivant, une sécurité indique la position de la valve.

Cette sécurité est composée d'un doigt palpeur 92, qui traverse sans frottement le corps tubulaire 9 et est en contact permanent avec la face terminale 17 du piston de la valve 6. Ce contact est établi sous l'effet de la poussée d'un ressort 96 qui, en tête du doigt palpeur 92, prend appui sur un bouchon fixé à la tête de connexion 12 d'une part, et sur une rondelle 93, munie d'un index 94, solidaire du doigt palpeur 92 d'autre part. Bien entendu, la force du ressort 96 est tarée pour ne pas ouvrir la valve par sa poussée.

Lorsque la valve 6 est fermée la longueur de la tige 92 est telle que l'index 94 ne touche pas le sommet de la pièce 12: on peut alors faire circuler sans danger le fluide aseptisant.

Si l'index 94 touchait le haut de la pièce 12 ce serait le signe d'un maintien en position basse du bas de la pièce 6, donc un risque de communication entre le liquide aseptisant et l'organisme existerait et demanderait vérification. Lorsque la valve 6 est ouverte, et le corps tubulaire 9 en position basse, l'index 94 du doigt 92 est, lui, en position haute, comme cela ressort des figures 2 et 3.

Cette sécurité mécanique peut être remplacée par une sécurité électrique dans le cas où, comme en figure 7, l'actionneur est un moteur électrique.

Cette sécurité électrique comprend des pièces communes avec la sécurité mécanique: doigt palpeur 92, rondelle 93, ressort 96, mais un contact électrique 33 permet de connaître la position du doigt 92. En service normal, lorsque le corps tubulaire 9 est en position haute, et la valve 6 fermée, la rondelle 93 doit être à mi-course. Si elle est en bout de course, en position basse, et écrase le contact électrique 33, cela signifie que la valve 6 est accidentellement ouverte: un circuit logique simple qui combine le contact 33 (position de la valve 6), le contact 30 (position du corps tubulaire 9) et une électrovanne sur le circuit de fluide aseptisant interdit de faire circuler ce fluide aseptisant.

Enfin les figures 15 et 16 montrent une sécurité directement installée sur le circuit d'arrivée des fluides aseptisants. Cette sécurité est particulièrement bien adaptée lorsque l'actionneur de la tête de raccordement est un actionneur fluidique, comprenant un piston 70. Le fluide aseptisant, avant d'arriver à l'un des tubes extérieures, 10 et 11, passe par une dérivation qui n'est ouverte que lorsque le corps tubulaire 9 est en position haute, donc en position asepsie-rinçage-débullage.

Le fluide aseptisant arrive par une tubulure 98, par exemple qui traverse le cylindre 71 de l'actionneur, et il repart, dans les mêmes conditions, par une autre tubulure 99. Les orifices de ces deux tubulures, repérés 96, débouchent en correspondance avec une gorge 97 usinée dans le piston 70 de l'actionneur. Lorsque le piston 70 est en position haute, la gorge 97 permet la circulation des fluides entre les deux tubulures 98 et 99. Lorsque le piston 70 est en position basse, valve ouverte, la gorge 97 n'est plus en face des orifices 96, et la paroi du piston 70 les obture hermétiquement: la circulation du fluide aseptisant est impossible.

Enfin, il a été dit que le corps tubulaire 9 comprend au moins deux canaux 20 et 21—les autres trous pour doigt palpeur, vis, doigt anti-rotation ne sont pas des canaux de circulation de fluides —et les figures n'en représentent que deux, pour ne pas obscurcir les dessins. Cependant appartient au domaine de l'invention le cas où un ou plusieurs canaux additifs sont forés dans le corps tubulaire 9. Ces canaux peuvent être utilisés, par exemple, pour injecteur des anticoagulants ou médicaments en même temps que le sang est réinjecté dans l'organisme après traitement.

Les matériaux de réalisation du dispositif selon l'invention n'ont pas été précisés jusqu'à présent. Tous les matériaux métalliques et/ou plastiques conviennent, sous les conditions

—que les pièces réalisées avec ces matériaux soient aseptisables et biocompatibles si elles sont en contact avec le sang ou un fluide injecté dans le réseau sanguin.

—que les matériaux répondent aux critères mécaniques qui assurent la fiabilité de la tête de raccordement, en particulier le bon glissement du corps tubulaire 9 dan le cylindre de guidage 8, et l'absence de corrosion en présence des fluides en contact avec les différentes parties de la tête de raccordement.

L'un des aspects caractéristiques de l'invention est de garantir une circulation efficace de divers fluides non seulement en vue d'aseptiser le système inamovible de passage percutané avant le début d'un traitement, mais aussi afin de balayer complètement les tubes et cavités, afin que des traces de désinfectant ou des bulles d'air ne restent pas dans le circuit au moment où débute un traitement.

C'est pour obtenir cette circulation fluide que la tête de raccordement est obligatoirement associée à une partie dite initiale, qui appartient au domaine de l'invention, située entre l'appareil de traitement—par exemple un dialyseur—et la tête de raccordement. Cette partie initiale est constituée par un dispositif distributeur de fluides, dont une caractéristique est—comme pour la tête de raccordement—de ne pas présenter de volume qui ne soit pas balayable par un fluide en mouvement.

La figure 17 représente un schéma très simplifié d'un système de traitement, comprenant une tête de raccordement 100, montée sur un passage percutané 101 implanté dans un organisme vivant, un boîtier distributeur 103+104 et un appareil de traitement ou de génération de fluide 102.

Le boîtier distributeur comporte obligatoirement deux parties séparées, dont chacune est reliée à l'un des deux tubes extérieurs 10 et 11 de la tête de raccordement. A titre d'exemple non limitatif, la partie 103 du boîtier distributeur est réunie par la connexion "a" au tube 10 et correspond à l'introduction de fluides dans la tête de raccordement 100, tandis que la partie 104 est réunie, par la connexion "b", au tube 11 et correspond à l'evacuation des fluides provenant de la tête de raccordement 100.

Aux deux tubes 10 et 11 situés en aval du boîtier distributeur 103+104 correspondent une pluralité de tubes situés en amont. Ces tubes, réunis au boîtier par les connexions "c, d, ... i, j", sont parcourus par les différents fluides délivrés ou absorbés par l'appariel de traitement 102: liquide désinfectant, sérum sanguin, produits médicamenteux etc. Il importe donc que le volume intérieur de chaque partie 103+104 du boîtier distributeur soit, tout comme la tête de raccordement, aseptisé et débullé avant traitement, et ne présente pas de volume mort, non balayable par un fluide.

Dans les cas simples où il n'y a dans le boîtier 103 par exemple que deux tubes en amont—par exemple "c" et "d"—et un tube en aval 10, une vanne boisseau à trois voies convient, puisqu'elle peut relier "c" à "a" ou "d" à "a" et parce qu'elle ne comporte aucun volume mort.

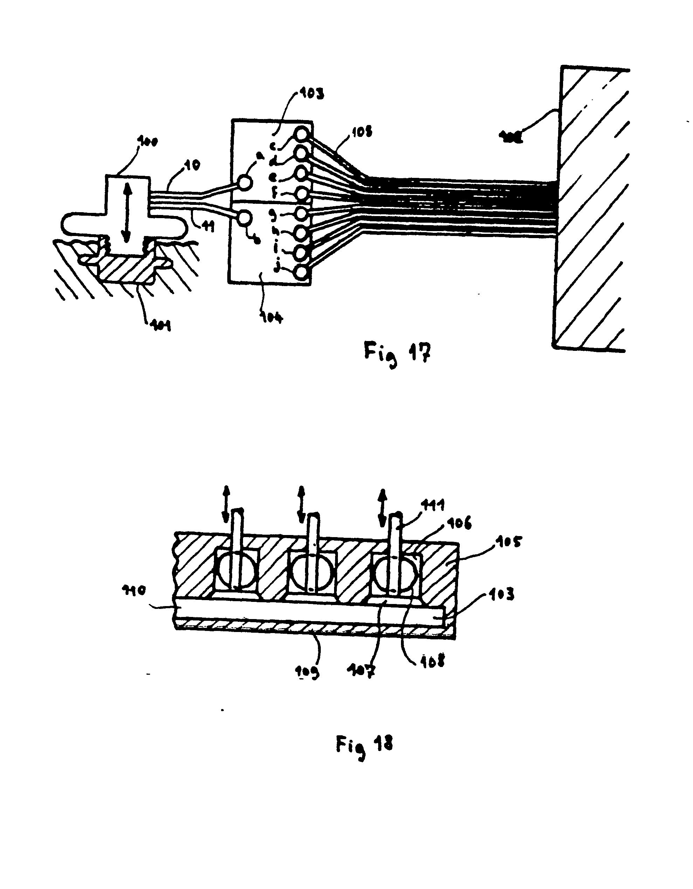

Dans le cas plus général, tel que celui schématisé en figure 17, il faut utiliser des vannes simples, maise montées sans volume mort nuisible. Un exemple, non limitatif, en est donné en figure 18.

En figure 18 est représentée une cavité 103, formant une moitié du boîtier distributeur, et comprise entre une pièce 105 et une plaque 109. La pièce 105 est usinée, de façon qu'y soient fermées une pluralité de chambres 106, chacune fermée du côté de la cavité 103 par une soupape 107, et alimentée en fluide par un orifice de tuyau 108. La surface de la cavité 103, fermée par les soupapes 107 et la surface de la pièce 105, ne présente aucune partie qui ne soit pas balayée par les fluides. Les soupapes 107 correspondent aux connexions "c, d, e, f" de la figure 17, et l'orifice libre 110 de la cavité 103 est relié à un tube extérieur, 10, de la tête de raccordement 100. On peut observer que les fluides parvenant à la cavité 103 par l'une des soupapes ou vannes c à f peuvent, selon le moment considéré d'un traitement complexe, comme la dialyse rénale par exemple, soit arriver en c et repartir en a vers la tête de raccordement 100, soit être interrompus, soit encore arriver en c et repartir en d, e ou f pour accompagner un fluide arrivant en a. On peut dont effectuer de cette façon dans le cas de la dialyse rénale une injection éventuelle d'anticoagulant soit vers la tête 100, soit vers l'appareil 102 afin de protéger l'ensemble des tuyaux de la coagulation.

Les soupapes 107 sont manoeuvrées de l'extérieur, soit manuellement, soit par un automatisme mécanique, électrique ou fluidique: leur mouvement longitudinal permet d'ouvrir ou fermer une vanne.

Bien entendu, le boîtier distributeur comporte des moyens de fixation, de raccordement ou d'étanchéité qui ne sont pas représentés de façon à simplifier la figure.

Dans le cas de la figure 18, il est toujours avantageux que le fluide le moins dangereux et le moins coagulable soit introduit ou éjecté par la vanne 111 qui est située au fond du cul-de-sac. Ainsi, il ne peut y avoir dans cette zone de stagnation prolongée de sang ou de liquide antiseptique par exemple. Il ne pourra stagner que du liquide de rinçage, lequel est sans inconvénient.

Le rôle des boîtiers 103 et 104 et son intégration dans le système comportant la tête de raccordement apparaîtra encore plus clairement si l'on considère que les diverses opérations d'asepsie, de rinçage, de débullage et d'anticoagulation éventuelle liées au problème du raccordement d'un réseau extérieur à un réseau intérieur à un organisme vivant doivent pouvoir s'effectuer de la manière la plus simple, la plus souple, la plus précise et la plus rapide possible.

Il ressort donc de toutes ces contraintes qu'il y a grand intérêt à ce que la longueur totale du circuit devant faire l'objet des circulations de fluids successifs pour asepsie, rinçage etc., à savoir le tuyau 10 plus le tuyau 11, soit la plus courte possible. Si c'est le cas, les quantités respectives de fluides nécessaires pour asepsie, rinçage etc. seront les plus faibles possibles et leur durée de passage sera de ce fait la plus courte possible. Il est de plus permis d'envisager pour certaines opérations complexes telles que la dialyse rénale par exemple une circulation de fluide momentanément en sens inverse du sens habituel.

Le lieu d'implantation idéal du boîtier 103+104 sera donc à proximité immédiate de la tête de raccordement 100 bien que cette remarque ne limite en rien l'invention.

Par exemple, si le passage percutané inamovible 101 est implanté sur le bras d'un malade, la tête de raccordement 100 étant fixée dessus, le boîtier 103+104 sera fixé sur ce même bras avec une sangle.

Les tubes 10 et 11 reliant la tête 100 aux boîtier 103+104 seront obligatoirement en matière assez souple afin que les efforts mécaniques éventuellement non négligeables causés par le raccordement aux boîtiers 103 et 104 du gros faisceau du tuyau et de fils provenant de l'appareil lourd 102 ne puissent pas se transmettre à la tête de raccordement.

La présence du boîtier 103+104 sur le corps du malade lui facilite par ailleurs la manoeuvre des vannes "a" à "j" quiy sont fixées dans le cas où celles-ci sont à commande manuelle.

Bien entendu, dans le cas où un appareillage d'ensemble pour une opération complexe telle que la dialyse rénale est partiellement ou totalement automatisé l'utilisation d'une têté de raccordement selon l'invention, munie d'une commande par fluide ou électrique confirmée avec l'utilisation, à proximité immédiate, d'un boîtier 103+104 selon l'invention comportant lui-même des commandes fluide ou électriques constitué un ensemble de dispositions indispensables qui complète l'ensemble des composants automatisés contenus dans l'appareil de traitement 102.

L'invention n'est pas limitée aux seules formes ou moyens décrits et représentés. Des forme équivalentes, ou des moyens différents mais évidents pour l'homme de l'art et dont la mise en oeuvre n'apporterait pas une activité inventive font partie de l'invention, dont la portée est précisée par les revendications suivantes.

**Revendications**

1. Dispositif inamovible de passage percutané d'accès au circuit sanguin d'un organisme vivant ou à toute zone de l'organisme, comprenant un puits d'accès formé par un corps creux (4) cylindrique ayant une extrémité interne d'accès au circuit sanguin munie d'une valve (6) ayant une position ouverte en communication avec le fluide sanguin et une position fermée interrompant ladite communication, et une extrémité externe comprenant des moyens de fixation amovible d'une tête de raccordement amovible, ladite tête de raccordement amovible comprenant des moyens d'échange de fluides avec le circuit sanguin comprenant un corps tubulaires (9) percé sur sensiblement tout sa longueur par au moins deux canaux (20, 21) non communiquants au moins lorsque la valve est en position ouverte, des moyens de manoeuvre de ladite valve comprenant un actionneur (13) étant prévus pour amener celle-ci en position ouverte, en communication avec le circuit sanguin, ou en position fermée interrompant ladite communication, une pièce de connexion (12) assurant la connexion entre chacun des canaux et un tube (10, 11) de raccordement extérieur, caractérisé en ce que ledit corps tubulaire (9) est percé sur toute sa longueur par au moins lesdits deux canaux (20, 21) débouchant dans le puits d'accès, lesdits moyens de manoeuvre (19) de la valve (6) laissant libre la circulation des fluides entre les canaux (20, 21) lorsque la valve (6) est fermée et assurant une interruption de la communication entre les canaux (20, 21) lorsque ladite valve est en position ouverte, en communication avec le circuit sanguin, et les moyens de fixation de la tête de raccordement amovible comprennent une embase (7), dont la partie centrale, en surépaisseur, est vissable sur ledit corps creux (4) et est percée pour former un cylindre de guidage (8), coaxial avec le corps creux (4).

2. Dispositif inamovible selon la revendication 1, caractérisé en ce que les moyens de manoeuvre (19) précités sont portés par l'extrémité du corps tubulaire (9) proche de la valve (6), des moyens d'actionnement (12) de la tête de raccordement amovible communiquant au corps tubulaire (9) au moins un mouvement de translation longitudinale, de sorte que le mouvement de translation du corps tubulaire (9) est capable d'amener la valve de ladite position fermée à ladite position ouverte et de mettre ainsi le circuit sanguin en communication avec les tubes extérieurs (10, 11).

3. Dispositif inamovible selon la revendication 1, caractérisé en ce que le moyen de manoeuvre de la valve (6) est constitué par une cloison (19), solidaire du corps tubulaire (9), placée entre les orifices des deux canaux (20, 21) cette cloison assurant un contact étanche avec les parois du puits d'accès et avec la valve, lorsque le corps tubulaire (9) est en position basse.

4. Dispositif inamovible selon la revendication 1, caractérisé en ce que le moyen de manoeuvre de la valve (6) est constitué par la prolongation (19) de l'un des deux canaux (21) par rapport à l'autre canal (20), le canal (21) prolongé s'obturant lorsqu'il entre en contact avec la surface du fond du puits d'accès (50) et ouvrant simultanément la valve (51, 52).

5. Dispositif inamovible selon la revendication 1, caractérisé en ce que l'actionneur est un actionneur mécanique, constitué par un écrou de manoeuvre (13, 80) maintenu à distance fixe par rapport à l'embase (7), cet écrou de manoeuvre (13, 80) étant muni d'un filetage qui coopère avec un filetage porté par la pièce de connexion (12, 70) la rotation de l'écrou de manoeuvre (13, 80) imprimant à la pièce de connexion (12, 70) et au corps tubulaire (9) un mouvement de translation longitudinale.

6. Dispositif inamovible selon la revendication 1, caractérisé, en ce que l'actionneur est un actionneur électrique, constitué par un stator (28) solidaire de l'embase (7) et un rotor (27) muni d'un filetage qui coopère avec un filetage porté par la pièce de connexion (12, 70), la rotation du rotor (27) imprimant à la pièce de connexion (12, 70) et au corps tubulaire (9) un mouvement de translation longitudinale.

7. Dispositif inamovible selon la revendication 1, caractérisé en ce que l'actionneur est un actionneur fluidique, activé par un gaz ou un liquide, constitué par un cylindre (71) solidaire du cylindre de guidage (8), et par un piston (70), solidaire du corps tubulaire (9) et formant pièce de connexion, le cylindre de l'actionneur fluidique étant fermé par un couvercle (79).

8. Dispositif inamovible selon la revendication 1, caractérisé en ce que la pièce de connexion est un piston (70), à parois lisses, passant à frottement doux dans un cylindre (71) à travers lequel sont connectés les tubes (10, 11) de raccordement extérieur, la connexion entre les canaux (20, 21) du corps tubulaire (9) et les tubes extérieurs (10, 11) se faisant au moyen d'évidements circulaires ou de rainures (63, 64) creusés par la paroi du cylindre (71).

9. Dispositif inamovible selon la revendication 1, caractérisé en ce que la pièce de connexion (70) est un piston (70), passant à frottement doux dans un cylindre (71) à travers lequel sont connectés les tubes (10, 11) de raccordement extérieur, la connexion entre les canaux (20, 21) du corps tubulaire (9) et les tubes extérieurs (10, 11) se faisant, au moyen d'évidements circulaires ou de rainures creusées parallèlement à l'axe dans la paroi du piston (70).

10. Dispositif inamovible selon l'une quelconque des revendications 8 ou 9, caractérisé en ce que, en vue de permettre une bonne circulation des fluides dans les gorges (59, 60, 63, 64), celles-ci sont en forme de U (74+76), la longueur des deux gorges parallèles (74, 76) étant égale à la longueur de translation de la pièce de connexion (70).

11. Dispositif inamovible selon la revendication 1, caractérisé en ce qu'il comporte en outre une sécurité mécanique de position, constituée par un doigt palpeur (92), passant longitudinalement à travers la pièce de connexion (12) et le corps tubulaire (9) ce doigt (92) étant maintenu au contact de a valve (6) sous l'action d'un ressort (96), et étant muni d'un index (94) qui indique la position (ouverte ou fermée) de la valve (6).

12. Dispositif inamovible selon l'une quelconque des revendications 6 ou 11, caractérisé en ce qu'il comporte en outre une sécurité électrique de position, constituée par deux contacts électriques (29, 30) de fin de course de la pièce de connexion (12) et par un contact électrique (33) de position du doigt palpeur (92), la combinaison de ces contacts (29+33 ou 30+33) autorisant ou interdisant le fonctionnement du moteur électrique (27+28).

13. Dispositif inamovible selon l'une quelconque des revendications 9 ou 10, caractérisé en ce qu'il comporte en outre une sécurité sur le circuit des fluides dangereux pour l'organisme dans lequel est implanté le passage percutané, cette sécurité étant constitué par une dérivation desdits fluides, au moyen des deux tubes (98, 99) connectés sur le cylindre (71) dans lequel se déplace la pièce de connexion en piston (70), une gorge (97) dans le piston (70) mettant en communication les orifices (96) desdits tubes (98, 99) lorsque le piston est en position haute (valve fermée), tandis que la paroi du piston obture lesdits orifices (96) lorsque le piston est en position basse (valve ouverte).

14. Système de distribution de fluides assurant la connexion des fluides entre les tubes extérieures (10, 11) d'une tête de raccordement et une pluralité de tubes (108) réunis à un appareil de traitement (102), caractérisé en ce qu'il comprend dispositif inamovible de passage percutané tel que defini à l'une quelconque des revendications 1 à 13, et un boîtier contenant autant de chambres étanches (103, 104) que de tubes reliés à la tête de raccordement et chacune de ces chambres étanches possède autant de vannes (107) sans volume mort que de tubes (108, 10 ou 11) qui y sont raccordés.

15. Système de distribution de fluides selon la revendication 14, caractérisé en ce qu'il est situé à proximité immédiate de la téte de raccordement et lui est relié par des liaisons souples uniquement.

16. Système selon la revendication 14, caractérisé en ce que toutes les manoeuvres de vannes (107) sont effectuables manuellement.

17. Système selon la revendication 14, caractérisé en ce que toutes les manoeuvres de vannes sont effectuables par commande électromagnétique, électrique ou fluidique obéissant, ainsi que la commande électrique ou fluidique de la tête de raccordement précitée à un séquentiel automatisant complètement l'opération de traitement du fluide concernant l'organisme.

18. Système de distribution de fluides selon la revendication 14, caractérisé en ce que les vannes sans volume mort sont constituées par des pièces en forme de soupape (107), affleurant la paroi de la cavité du boîtier (103).

**Patentansprüche**

1. Nicht lösbare Perkutandurchlaßvorrichtung für den Zutritt zum Blutkreislauf eine lebenden Organismus oder zu jeder anderen Zone des Organismus, umfassend einen Eintrittsschacht, der durch einen zylindrischen Höhlkörper (4) gebildet ist, dessen inneres Zutrittsende zum Blutkreislauf mit einem Ventil (6) mit einer in Kommunikation mit dem Blutfluid stehenden, offenen Position und einer diese Kommunikation unterbrechenden, geschlossenen Position versehen ist und dessen äußeres Ende Mittel zur lösbaren Befestigung eines lösbaren Verbindungskopfes umfaßt, wobei der lösbare Verbindungskopf Mittel zum Austausch von Fluiden mit dem Blutkreislauf mit einem rohrförmigen Körper (9), der im wesentlichen über seine gesamte Länge von mindestens zwei zumindest in der offenen Position des Ventils nicht kommunizierenden Kanälen (20, 21) durchsetzt ist, Mittel zum Betätigen des Ventils mit einem Betätigungsorgan (13) zum Bewegen des Ventils in die geöffnete Position in Kommunikation mit dem Blutkreislauf oder in die die Kommunikation unterbrechende, geschlossene Position und ein die Verbindung zwischen jedem der Kanäle und einem Außenverbindungsrohr (10, 11) sicherndes Verbindungsstück (12) umfaßt, dadurch gekennzeichnet, daß der rohrförmige Körper (9) über seine gesamte Länge mindestens von den beiden Kanälen (20, 21), die in den Zutrittsschacht münden, durchsetzt ist, wobei die Mittel (19) zur Betätigung des Ventils (6) die Zirkulation der Fluide zwischen den Kanälen (20, 21) bei geschlossenem Ventil (6) ungehindert lassen und bei in geöffneter Position befindlichem und mit dem Blutkreislauf kommunizierendem Ventil eine Unterbrechung der Kommunikation zwischen den Kanälen (20, 21) sichern, und die Mittel zur Befestigung des lösbaren verbindungskopfes ein Ansatzstück (7) umfassen, dessen zentraler Teil mit einer Überdicke auf den Hohlkörper (4) aufschraubbar und zuŕ Bildung eines zum Hohlkörper (4) koaxialen Führungszylinders (8) durchbohrt ist.

2. Nicht lösbare Vorrichtung nach Anspruch 1, dadurch gekennzeichnet, daß die Betätigungsmittel (19) am dem Ventil (6) nahen Ende des rohrförmigen Körpers (9) vorgesehen sind, wobei Mittel zum Betätigen (12) des lösbaren Verbindungskopfes dem rohrförmigen Körper (9) mindestens eine Verschiebebewegung in Längsrichtung aufdrükken, sodaß die Verschiebebewegung des rohrförmigen Körpers (9) das Ventil aus der geschlossenen Position in die geöffnete Position bringen und so den Blutkreislauf in Kommunikation mit den Außenrohren (10, 11) setzen kann.

3. Nicht lösbare Vorrichtung nach Anspruch 1, dadurch gekennzeichnet, daß das Mittel zur Betätigung des Ventiles (6) durch eine mit dem rohrförmigen Körper (9) verbundene und den Öffnungen der beiden Kanäle (20, 21) zwischengeschaltete Zwischenwand (19) gebildet ist, welche Zwischenwand in der tiefstehenden Position des rohrförmigen Körpers (9) den dichten Kontakt zwischen den Wänden des Zutrittsschachtes und dem Ventil gewährleistet.

4. Nicht lösbare Vorrichtung nach Anspruch 1, dadurch gekennzeichnet, daß das Mittel zur Betätigung des Ventils (6) durch eine Verlängerung (19) einer der beiden Kanäle (21) in bezug auf den anderen Kanal (20) gebildet ist, wobei der verlängerte Kanal (21) bei Inkontakttreten mit der Oberfläche des Bodens des Zutrittsschachtes (50) angesperrt und gleichzeitig das Ventil (51, 52) geöffnet wird.

5. Nicht lösbare Vorrichtung nach Anspruch 1, dadurch gekenneichnet, daß das Betätigungsorgan ein mechanisches Betätigungsorgan ist, welches durch eine in bestimmtem Abstand von Ansatzstück (7) gehaltene Betätigungsmutter (13, 80) gebildet ist, welche Betätigungsmutter (13, 80) mit einem Gewinde versehen ist, das mit einem am Verbindungsstück (12, 70) vorgesehenen Gewinde zusammenwirkt, wobei die Drehung der Betätigungsmutter (13, 80) eine Verschiebebewegung Längsrichtung auf das Verbindungsstück (12, 70) und auf den rohrförmigen Körper (9) aufdrückt.

6. Nicht lösbare Vorrichtung nach Anspruch 1, dadurch gekennzeichnet, daß das Betätigungsorgan ein elektrisches Betätigungsorgan ist, welches durch einen mit dem Ansatzstück (7) verbundenen Stator (28) und einen Rotor (27) gebildet ist, der mit einem Gewinde versehen ist, welches mit einem am Verbindungsstück (12, 70) vorgesehenen Gewinde zusammenwirkt, wobei die Rotation des Rotors (27) eine Verschiebebewegung in Längsrichtung auf das Verbindungsstück (12, 70) und auf den rohrförmigen Körper (9) aufdrückt.

7. Nicht lösbare Vorrichtung nach Anspruch 1, dadurch gekennzeichnet, daß das Betätigungsorgan ein durch ein Gas oder eine Flüssigkeit aktiviertes Fluidbetätigungsorgan ist, welches durch einen mit dem Führungszylinder (8) verbundenen Zylinder (71) und durch einen mit dem rohrförmigen Körper (9) verbundenen und ein Verbindungsstück bildenden Kolben (70) gebildet ist, wobei der Zylinder des Fluidbetätigungsorgans durch einen Deckel (79) verschlossen ist.

8. Nicht lösbare Vorrichtung nach Anspruch 1, dadurch gekennzeichnet, daß das Verbindungsstück ein Kolben (70) mit glatten Wänden ist, der mit leichter Reibung in einem Zylinder (71) gleitet, über den die Außenverbindungsrohre (10, 11) verbunden sind, wobei die Verbindung zwischen den Kanälen (20, 21) des rohrförmigen Körpers (9) und den Außenrohren (10, 11) mittels kreisförmiger Ausnehmungen oder mittels in der Wand des Zylinders (71) ausgehöhlter Rillen (63, 64) erfolgt.

9. Nicht lösbare Vorrichtung nach Anspruch 1, dadurch gekennzeichnet, daß das Verbindungsstück (70) ein Kolben (70) ist, der mit leichter Reibung in einem Zylinder (71) gleitet, über den die Außenverbindungsrohre (10, 11) verbunden sind, wobei die Verbindung zwischen den Kanälen (20, 21) des rohrförmigen Körpers (9) und den Außenrohren (10, 11) mittels kreisförmige Ausnehmungen oder mittels parallel zur Achse in der Wand des Zylinders (70) ausgehöhlter Rillen erfolgt.

10. Nicht lösbare Vorrichtung nach einem der Ansprüche 8 oder 9, dadurch gekennzeichnet, daß zur Ermöglichung einer guten Zirkulation der Fluide in den Kanälen (59, 60, 63, 64), letztere U-förmig (74+76) sind, wobei die Länge der beiden parallelen Kanäle (74, 76) gleich der Verschiebungslänge des Verbindungsstücks (70) ist.

11. Nicht lösbare Vorrichtung nach Anspruch 1, dadurch gekennzeichnet, daß sie weiters eine mechanische Positionssicherung umfaßt, bestehend aus einem Fühlstift (92), der der Länge nach durch das Verbindungsstück (12) und den rohrförmigen Körper (9) geht, welcher Stift (92) unter der Wirkung einer Feder (96) mit dem Ventil (6) in Kontakt gehalten und mit einem Anzeiger (94) zum Anzeigen der (geöffneten oder geschlossenen) Position des Ventils (6) versehen ist.

12. Nicht lösbare Vorrichtung nach einem der Ansprüche 6 oder 11, dadurch gekennzeichnet, daß sie weiters eine elektrische Positionssicherung umfaßt, bestehend aus zwei elektrischen Endkontakten (29, 30) des Verbindungsstücks (12) und aus einem elektrischen Positionskontakt (33) des Fühlstiftes (92), wobei die Kombination dieser Kontakte (29+33 oder 30+33) den Betrieb des Elektromotors (27+28) zuläßt oder versagt.

13. Nicht lösbare Vorrichtung nach einem der Ansprüche 9 oder 10, dadurch gekennzeichnet, daß sie weiters eine Sicherung am Kreislauf der für den Organismus, in dem der Perkutandurchlaß implantiert ist, gefährlichen Fluide umfaßt, welche Sicherung aus einer Ableitung dieser Fluide mittels zweier Rohre (98, 99) besteht, die an den Zylinder (71), in dem sich das Kolben-Verbindungsstück (70) bewegt, angeschlossen sind, wobei ein Kanal (97) im Kolben (70) in hochgestellter Position des Kolbens (Ventil geschlossen) die Öffnungen (96) der Rohre (98, 99) in Kommunikation setzt, während die Wand des Kolbens in tiefgestellter Position des Kolbens (Ventil geöffnet) die Öffnungen (96) verschließt.

14. System zur Verteilung von Fluiden, welches die Verbindung der Fluide zwischen den Außenrohren (10, 11) eines Verbindungskopfes und einer Mehrzahl von Rohren (108) gewährleistet, die an einem Behandlungsapparat (102) zusammenlaufen, dadurch gekennzeichnet, daß es eine nicht lösbare Perkutandurchlaßvorrichtung nach einem der Ansprüche 1 bis 13 und einen Kasten, der so viele dichte Kammern (103, 104) enthält, so viele Rohre an den Verbindungskopf angeschlossen sind, umfaßt und jede dieser dichten Kammern so viele Ventile (107) ohne Totvolumen besitzt, so viele Rohre (108, 10 oder 11) dort angeschlossen sind.

15. System zur Verteilung von Fluiden nach Anspruch 14, dadurch gekennzeichnet, das es in unmittelbarer Nähe des Verbindungskopfes angeordnet und mit diesem ausschließlich über nachgiebige Verbindungen verbunden ist.

16. System nach Anspruch 14, dadurch gekennzeichnet, daß sämtliche Ventilbetätigungen (107) händisch ausführbar sind.

17. System nach Anspruch 14, dadurch gekennzeichnet, daß sämtliche Ventilbetätigungen mittels elektromagnetischer, elektrischer oder Fluid-Steuerung ausführbar sind, welche, so wie die elektrische oder Fluid-Steuerung des Verbindungskopfes, einer Sequenz gehorchen, die den Schritt der Behandlungs des den Organismus betreffenden Fluids vollkommen automatisch ausführt.

18. System zur Verteilung von Fluiden nach Anspruch 14, dadurch gekennzeichnet, daß die Ventile ohne Totvolumen durch Stücke in Ventilform (107) gebildet sind, die bündig in der Wand des Hohlraums des Kastens (103) eingelassen sind.

**Claims**

1. Irremovable device for percutaneous access into the blood circulatory system of a living body, or to any part of the body, comprising a passageway formed by a hollow cylindrical piece (4) having an internal end for access into the blood circulatory system equipped with a valve (6) which has an open position of communication with the blood system and a closed position closing off said communication, and an external end comprising removable means for fixing a removable connecting head, said removable connecting head comprising means for exchanging fluids with the blood system comprising a tubular body (9) which is traversed through substantially its entire length by at least two channels (20, 21) which are non-communicating at least when the valve is in open position, means for operating said valve comprising an actuator (13) being provided to bring said valve, in open position, in communication with the blood system, or in closed position, interrupting said communication, a connection piece (12), ensuring the connection between each one of the channels and an external connecting tube (10, 11), characterized in that said tubular body (9) is traversed through its entire length by at least said two channels (20, 21) issuing into the access passageway, said means (19) for operating the valve allowing the circulation of the fluids between the channels (20, 21) when valve (6) is closed and obturating the communication between the channels (20, 21) when said valve is in open position, communicating with the blood system, and the means for fixing the removable connecting head comprise a seating (7) of which the center part, being thicker, is screwable on said hollow piece (4) and is perforated to form a guiding cylinder (80, coaxial with the hollow body (4).

2. Irremovable device according to claim 1, characterized in that said operating means (19) are carried by the end of the tubular body (9) which is near the valve (6), means (12) for actuating the removable connecting head imparting to the tubular body (9) at least a longitudinal translation motion, so that the translatory movement of the tubular body (9) is capable of bringing the valve from said closed position to said open position thus placing the blood system in communication with the external tubes (10, 11).

3. Irremovable device according to claim 1, characterized in that the means for operating the valve (6) are constituted by a wall (19), fast with the tubular body (9), placed between the orifices of the two channels (20, 21), said wall ensuring tight contact with the walls of the passageway and with the valve when the tubular body (9) is in low position.

4. Irremovable device according to claim 1, characterized in that the means for operating the valve (6) are constituted by the extension (19) of one (21) of the two channels with respect to the other channel (20), extended channel (21) being obturated when it comes into contact with the surface of the bottom of the passageway (50) and simultaneously opening valve (51, 52).

5. Irremovable device according to claim 1, characterized in that the actuator is a mechanical actuator, constituted by an operating nut (13, 80) held at fixed distance with respect to the seating (7), said operating nut (13, 80) being provided with a thread which cooperates with a thread provided on the connecting piece (12, 70), the rotation of the rotor (27) imparting to the connecting piece (12, 70) and to the tubular body (9) a movement of longitudinal translation.

6. Irremovable device according to claim 1, characterized in that the actuator is an electrical actuator, constituted by a stator (28) fixed to the seating (7) and a rotor (27) provided with a thread which cooperates with a thread provided on the connecting piece (12, 70), the rotation of the rotor (27) imparting to the connecting piece (12, 70) and to the tubular body (9) a movement of longitudinal translation.

7. Irremovable device according to claim 1, characterized in that the actuator is a fluidic actuator activated by a gas or a liquid, constituted by a cylinder (71) fast with the guiding cylinder (8), and by a piston (70), fast with the tubular body (9) and acting as a connecting piece, the cylinder of the fluidic actuator being closed off by a cap (79).

8. Irremovable device according to claim 1, characterized in that the connecting piece is a piston (70) with smooth walls moving with mild friction in a cylinder (71) through which are connected the external connecting tubes (10, 11), the connection between the channels (20, 21) of the tubular body (9) and the external tubes (10, 11) being achieved by means of circular recesses or grooves (63, 64) formed by the wall of the cylinder (71).

9. Irremovable device according to claim 1, characterized in that the connecting piece (70) is a piston (70), sliding with mild friction in a cylinder (71) through which are connected the external connecting tubes (10, 11), the connection between the channels (20, 21) of the tubular body (9) and the external tubes (10, 11) being achieved by means of circular recesses or grooves formed in parallel to the axis in the wall of the piston (70).

10. Irremovable device according to any one of claims 8 or 9, characterized in that, in order to allow a good circulation of the fluids inside the grooves (59, 60, 63, 64), said grooves are U-shaped (74+76), the length of the two parallel grooves (74, 76) being equal to the length of translation of the connecting piece (70).

11. Irremovable device according to claim 1, characterized in that it further comprises a mechanical position locking system, constituted by a contact finger (92), traversing longitudinally the connecting piece (12) and the tubular body (9), said finger (92) being held in contact with the valve (6) by the action of a spring (96), and being equipped with a pointer (94) indicating the (closed or open) position of the valve (6).

12. Irremovable device according to any one of claims 6 or 11, characterized in that it further comprises an electric position locking system, constituted by two electrical switches (29, 30) indicating the limit of displacement for the connecting piece (12) and by an electrical position switch (33) for the contact finger (92), the combination of said switches (29+33 or 30+33) allowing or preventing the operation of the electric motor (27+28).

13. Irremovable device according to any one of claims 9 or 10, characterized in that it further comprises a safety device on the circuit of fluids dangerous for the system in which the percutaneous access device is implanted, said safety device being constituted by a diverting system for said fluids, composed of the two tubes (98, 99) connected on the cylinder (71) in which the connecting piece moves as a piston (70) a groove (97) in the piston (70) allowing the orifices (96) to communicate with said tubes (98, 99) when the piston is in high position (valve closed), while the wall of the piston closes off said orifices (96) when the piston is in low position (valve open).

14. Systems for the distribution of fluids ensuring connection of the fluids between the external tubes (10, 11) of a connecting head and a plurality of tubes (108) connected to a treatment apparatus (102), characterized in that it comprise an irremovable percutaneous access device such as defined in any one of claims 1 to 13, and a housing containing as many tight chambers (103, 104) as there are tubes connected to the connecting head, each of said chambers having as many valves (107) without lost volume as there are tubes (108, 10 or 11) connected thereto.

15. Fluid distribution system according to claim 14, characterized in that it is situated in the immediate vicinity of the connecting head and is joined thereto by flexible connection only.

16. System according to claim 14, characterized in that all the valves (107) can be operated by hand.

17. System according to claim 14, characterized in that all the valves can be operated by electromagnetic, electric and fluidic control working, as the electric or fluidic control of said connecting head, according to a sequential which completely automatizes the fluid treatment operation in the blood system.

18. Fluid distribution system according to claim 14, characterized in that the valves without lost volume are constituted by valve-shaped pieces (107), which are flush with the wall of the cavity of housing (103).

EP 0 207 817 B1

Fig. 1

Fig. 2

1

Fig. 3

Fig. 4

Fig. 5

Fig. 6

Fig. 7

Fig. 8

Fig. 9

Fig. 10

Fig. 11

Fig 13

Fig. 12

Fig. 14

Fig. 15

Fig. 16

Fig 17

Fig 18